# EUROPÄISCHE PATENTSCHRIFT

(11) **EP 1 894 525 B1**
(45) Veröffentlichungstag und Bekanntmachung des Hinweises auf die Patenterteilung: **07.03.2012**
(21) Anmeldenummer: 06120033.3
(22) Anmeldetag: 04.09.2006
(51) Int. Cl.: A61B 5/15, A61B 17/06, A61B 19/02, B65D 85/24

(54) **Verpackung für hydrophile medizinische Instrumente**
Packaging for hydrophilic medical instruments
Emballage pour instruments médicaux hydrophiliques

(43) Veröffentlichungstag der Anmeldung: 05.03.2008
(73) Patentinhaber: F. Hoffmann-La Roche AG, 4070 Basel (CH); Roche Diagnostics GmbH, 68305 Mannheim (DE)
(72) Erfinder: Niederberger, Brigitte, 8800 Thalwil (CH); Zimmer, Volker, 67299 Laumersheim (DE)
(74) Vertreter: Stößel, Matthias

(56) Entgegenhaltungen:
- WO-A-2005/104948
- US-A1- 2006 030 788

## Beschreibung

Die Erfindung bezieht sich auf eine Verpackung aus Kunststoff für hydrophile medizinische Instrumente, insbesondere für hydrophil beschichtete Lanzetten, welche die Hydrophilie des umschlossenen Gegenstands über einen langen Zeitraum hinweg sicherstellt.

In der medizinischen Diagnostik ermöglicht die Untersuchung von Blutproben oder von interstitiellen Flüssigkeiten das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung dieser Probe kann die Haut beispielsweise an der Fingerbeere oder dem Ohrläppchen der zu untersuchenden Person mit Hilfe einer sterilen, scharfen Lanzette perforiert werden, um hierdurch einige wenige Mikroliter Blut für die Analyse zu gewinnen. Insbesondere eignet sich diese Methode der Probenentnahme für die Analyse einer Probe, die unmittelbar nach der Probengewinnung durchgeführt wird.

Insbesondere im Bereich des so genannten "Home-Monitorings", also dort, wo medizinische Laien selbst einfache Analysen des Blutes oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (so genannte Stechhilfen), angeboten. Diese Stechhilfen ermöglichen insbesondere dem medizinischen Laien eine möglichst schmerzarme und reproduzierbare Probengewinnung. Derartige Lanzetten und Gerätschaften sind beispielsweise Gegenstand von WO-A 98/48695, US 4,442,836 oder US 5,554,166.

Die eigenhändige, häufig in Privathaushalten durchgeführte Blutzuckerbestimmung, ist heute eine weltweit verbreitete Methode in der Diabetes-Kontrolle. Blutzuckermessgeräte im Stand der Technik bestehen aus einem Analysegerät, in das ein Testelement (Teststreifen) eingeführt wird. Das Testelement wird mit einem Tropfen einer Probe in Kontakt gebracht, der kurz zuvor mittels einer Stechhilfe beispielsweise aus der Fingerbeere gewonnen wurde.

Die zahlreichen Systemkomponenten (Lanzette, Stechhilfe, Testelement und Analysegerät) benötigen viel Platz und bedingen eine relativ komplexe Handhabung. Mittlerweile gibt es jedoch Systeme mit einem höheren Grad an Integration und somit einer einfachen Handhabung, bei denen beispielsweise die Testelemente im Analysegerät magaziniert und für die Messung zur Verfügung gestellt werden. Der nächste Schritt der Miniaturisierung ist beispielsweise die Integration von mehreren Funktionen bzw. Funktionselementen in einem einzigen analytischen Hilfsmittel (Disposable). Durch geeignete Kombination von Stechvorgang und sensorischer Analyse auf einem Testelement lässt sich der Bedienablauf deutlich vereinfachen.

Im Stand der Technik ist beispielsweise bekannt, die Lanzetten derartiger analytischer Hilfsmittel mit einer Kapillarstruktur zu versehen oder sie zu einem Teil einer Kapillarstruktur auszubilden (siehe hierzu WO 2005/104948 A1). In diesem Falle wird die Lanzettenspitze nach dem Einstich und dem Probenaustritt an die Probe herangeführt, um die Probe mit Hilfe der Kapillare zu sammeln.

Die Lanzettenspitze zum Sammeln von Blut bzw. interstitieller Flüssigkeiten wird typischerweise im Voraus sterilisiert und durch einen Sterilschutz (z.B. in Form einer Kappe oder Tasche) in einem sterilen Zustand gehalten, bevor sie für einen Stechvorgang verwendet wird. Hierdurch wird sichergestellt, dass die Lanzettenspitze durch die Umgebung nicht kontaminiert wird. Ferner werden auch Vorkehrungen dafür getroffen, dass die Lanzettenspitze nach einem erfolgten Stechvorgang abgeschirmt wird (gegebenenfalls durch dieselbe Kappe oder Tasche), so dass unbeabsichtigte Verletzungen und damit einhergehende Infektionen durch an der Lanzettenspitze anhaftende Flüssigkeiten vermieden werden.

Bei Einzellanzetten kann beispielsweise ein Sterilschutz hergestellt werden, indem die Spitze der Lanzette in einem Arbeitsgang mit der Erzeugung des Lanzettenkörpers mit Kunststoff umspritzt wird. Vor Gebrauch entfernt der Anwender dieses Teil manuell, meist beim Einsetzen der Lanzette in die Stechhilfe. Im Fall von magazinierten Lanzetten sind ähnliche Sterilschutzeinrichtungen gebräuchlich, beispielsweise solche, bei denen die Lanzette nach hinten aus einem sterilen Schutz herausgezogen wird, woraufhin der Sterilschutz per Federkraft aus dem Stichweg befördert wird. Hierzu sind relativ aufwendige Mechaniken erforderlich, insbesondere Federn, die in das Verbrauchsmaterial integriert sind.

Im Stand der Technik sind zahlreiche Verpackungen für die vorstehend beschriebenen medizinischen Hilfsmittel beschrieben.

EP 1 360 935 A1 beschreibt eine bandförmige Verpackung für eine Vielzahl von medizinischen Hilfsmitteln. Die Hilfsmittel sind in Vertiefungen eines ersten Bandabschnitts untergebracht, die mit einem zweiten Bandabschnitt bedeckt sind. Um die Sterilität der medizinischen Hilfsmittel zu gewährleisten, sind diese in einem flexiblen Verpackungsmaterial eingeschweißt.

DE 28 03 345 B1 beschreibt ein Blutentnahmegerät mit einer unter Krafteinwirkung gegen die Köperoberfläche eines Patienten bewegbaren Nadel. Die Nadeln sind hierbei in so genannten Streifenpackungen mit nebeneinander liegenden Taschen bevorratet. Die Streifenpackungen werden aus einer Grundfolie und einer Deckfolie aus Papier, Kunststofffolie oder dergleichen gebildet, wobei Grund- und Deckfolie aufeinander gelegt und in einem leiterförmigen Bereich unter Bildung von die Blutlanzetten allseits umschließenden Taschen miteinander verbunden werden.

WO 2004/075760 A1 betrifft eine automatisch öffnende Verpackung für medizinische Hilfsmittel, die einen oberen flexiblen Flächenabschnitt, der ein distales Ende, ein proximales Ende, einen ersten peripheren Rand und einen zweiten peripheren Rand aufweist, einen rundherum flexiblen Flächenabschnitt, der ein distales Ende, ein proximales Ende, einen ersten peripheren Rand und einen zweiten peripheren Rand aufweist, umfasst. Hierbei sind der obere und der untere flexible Flächenabschnitt dazu geeignet, dass sie entlang zumindest eines Segments ihrer ersten und zweiten peripheren Ränder lösbar miteinander versiegelt werden können, wodurch ein medizinisches Gerät innerhalb des oberen und des unteren flexiblen Flächenabschnitts umschlossen wird. An dem distalen Ende des oberen flexiblen Flächenabschnitts und an dem distalen Ende des unteren flexiblen Flächenabschnitts ist ein Bund angebracht, wobei der Bund an den distalen Enden des unteren und oberen flexiblen Flächenabschnitts in einer Weise angebracht ist, dass eine relative, abgleitende Bewegung des Bundes und der proximalen Enden des oberen und des unteren flexiblen Flächenabschnitts, die einen Abstand dazwischen verringert, dazu führt, dass sich ein Auseinanderziehen des oberen und des unteren flexiblen Flächenabschnitts ergibt. Hierdurch wird automatisch die Verpackung geöffnet und zumindest ein Abschnitt des medizinischen Geräts freigegeben.

Ferner offenbart die WO 2005/104948 A1 ein Testmagazin mit zwei sandwichartig miteinander verbundenen, aufwickelbaren Folienbändern, zwischen denen Aufnahmezellen für Testelemente freigehalten sind, welche jeweils eine Stecheinheit zum Einstechen in Körpergewebe und eine Testeinheit zur Beaufschlagung mit Körperflüssigkeit umfassen.

Lanzetten und insbesondere Mikronadeln mit Kapillaren sind üblicherweise aus chirurgischem Stahl, wie beispielsweise einem chromlegierten, nichtrostenden Vergütungsstahl mit einem niedrigen Kohlenstoffgehalt (Werkstoffnummer 1.4021) gefertigt, was für die Entnahme von Blut und interstitiellen Flüssigkeiten mittels Kapillarkräften ein Problem darstellen kann. Die Ursache hierfür liegt in der Hydrophobizität des Stahls begründet, welche eine Füllung der Kapillare mittels Kapillarkräften zumindest teilweise verhindert. Um dennoch eine beinahe vollständige oder vollständige Füllung der Kapillare zu erreichen, wird die Kapillare üblicherweise hydrophil, insbesondere mit einer Schicht aus SiO₂, TiO₂, Polyacrylsäure oder Dextransulfat beschichtet. Die hydrophilen Oberflächen sind hoch energetisch, was dazu führt, dass diese durch Absorption von Gasmolekülen an ihre Oberfläche sukzessiv ihre Oberflächenenergie reduzieren, was im Falle der Absorption von apolaren Verbindungen schrittweise zu einem Verlust der Hydrophilie der Beschichtung führt. Der vorstehend beschriebene Stand der Technik gibt keinen Hinweis darauf, die Hydrophilie der Oberflächen von Lanzetten, Stechhilfen und medizinischen Hilfsmitteln möglichst über einen langen Zeitraum hinweg zu gewährleisten.

Aufgabe der vorliegenden Erfindung ist es daher, die Nachteile des Standes der Technik zu vermeiden und insbesondere eine Verpackung für hydrophile Gegenstände wie hydrophile medizinische Instrumente, Mikronadeln mit Kapillaren, Lanzetten und medizinische Hilfsmittel zur Verfügung zu stellen, welche die Hydrophilie des von der Verpackung umschlossenen Gegenstands dauerhaft gewährleistet.

Diese Aufgabe wird erfindungsgemäß gelöst durch eine Verpackung aus Kunststoff mit zumindest einer Aufnahmezelle mit zumindest einem teilweise hydrophilen medizinischen Instrument zum Einstechen in Körpergewebe, die nach der Strahlungssterilisation einen Anteil an flüchtigen Bestandteilen von ≤ 0,1 µg/cm², bevorzugt von ≤ 0,01 µg/cm² aufweist. Es hat sich nämlich gezeigt, dass durch die Verminderung der von dem Verpackungsmaterial freigesetzten leichtflüchtigen Bestandteile auf Konzentrationen ≤ 0,1 µg/cm² eine dauerhafte Funktionalität der mit einer hydrophilen Schicht versehenen medizinischen Instrumente gewährleistet werden kann. Unter dem Begriff "dauerhaft" ist in dem vorliegenden Fall zu verstehen, dass das hydrophil beschichtete medizinische Instrument zumindest nach etwa dreimonatiger Lagerung in der erfindungsgemäßen Verpackung identische Benetzungseigenschaften wie vor seiner Lagerung aufweist.

Erfindungsgemäß ist unter dem Begriff "Verpackung aus Kunststoff" eine Umhüllung aus Kunststoff zu verstehen, welche das darin eingehüllte medizinische Instrument vor äußeren Verunreinigungen und Unsterilitäten schützt. Somit soll diese eine möglichst dauerhafte Sterilität des Instruments gewährleisten. Erfindungsgemäß ist die Verpackung aus Kunststoff gefertigt und besteht somit zu 100% aus Kunststoff. In einer Ausführungsform der Erfindung umfasst die Verpackung einen Kunststoff oder ein Kunststoffgemisch als Verpackungsmaterial neben weiteren Materialien, wie beispielsweise Metallfolien. Dabei kann der Anteil an Kunststoff in weiten Grenzen schwanken und variiert werden, beispielsweise im Bereich von 90 Gew.-% : 10 Gew.-% bis 10 Gew.-% : 90 Gew.-%.

Unter dem Begriff medizinisches Instrument sind gemäß der Erfindung Stechhilfen, Lanzetten, Mikronadeln, besonders bevorzugt Mikronadeln mit einer Kapillare und gleichwirkende medizinische Hilfsmittel zu verstehen. Derartige medizinische Instrumente werden vorzugsweise zur Entnahme von Blut oder interstitiellen Flüssigkeiten verwendet.

Unter dem Begriff "flüchtige Bestandteile" sind Bestandteile der Verpackung zu verstehen, welche sukzessiv von dieser freigesetzt werden. Das Wort "flüchtig" impliziert hierbei, dass die zur Gruppe der leicht flüchtigen Bestandteile zählenden Stoffe aufgrund ihres hohen Dampfdrucks bzw. niedrigen Siedepunkts verdampfen. Bei den flüchtigen Bestandteilen handelt es sich beispielsweise um Weichmacher wie Phthalate. Die flüchtigen Bestandteile lassen sich mittels Headspace-Gaschromatographie charakterisieren und quantifizieren.

Erfindungsgemäß ist die Verpackung aus Kunststoff eine Verpackung, die ein medizinisches Instrument, vorzugsweise eine Lanzette zum Einstechen in Körpergewebe umhüllt. Das medizinische Instrument ist ausgewählt aus der Gruppe der Stechhilfen, Lanzetten, Mikronadeln, besonders bevorzugt Mikronadeln mit einer Kapillare und medizinischen Hilfsmitteln.

Um die Aufnahme von Blut bzw. interstitiellen Flüssigkeiten zu verbessern, sind die vorstehend beschriebenen medizinischen Instrumente zumindest teilweise hydrophil und bevorzugt zumindest abschnittsweise mit einer hydrophilen Beschichtung versehen. Die abschnittsweise hydrophile Beschichtung ist insbesondere an den Stellen aufgebracht, die bei der Probenentnahme in Kontakt mit Blut bzw. interstitieller Flüssigkeit kommen. Hierdurch wird in vorteilhafter Weise erreicht, dass sich beispielsweise auch beschichtete Kapillaren aus Chirurgenstahl beinahe vollständig oder vollständig mit der Testflüssigkeit füllen.

In einer Ausführungsform der Erfindung umfasst die Verpackung Polyester, insbesondere Polyethylenterephthalat (PET) als Verpackungsmaterial neben weiteren Materialien. Dabei kann der Anteil an Polyester in weiten Grenzen schwanken und variiert werden, beispielsweise im Bereich von 100:1 bis 1:100. In einer weiteren Ausführungsform besteht die Verpackung im Wesentlichen aus Polyester, d.h., dass neben Polyester noch ein oder mehrere weitere Materialien vorhanden sein können, diese aber nur in sehr geringen Mengen unter 1 Gew.-% vorliegen. In einer weiteren Ausführungsform kann die Verpackung aus Polyester bestehen, also einen Anteil von 100 % Polyester aufweisen.

Polyester ist die Bezeichnung für Polymere der allgemeinen Formel I beziehungsweise II: 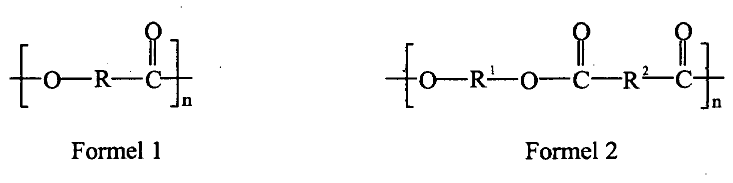 siehe hierzu Römpp, Chemie Lexikon, Thieme Verlag Stuttgart, 10., erweiterte Auflage, 1996, Band Pl-S, Seiten 3527 bis 3528.

Polyester werden durch Ringöffnungsreaktionen von Lactonen (I) oder durch Polykondensation von Hydroxycarbonsäuren (I) bzw. von Diolen und Carbonsäure(-Derivaten) (II) hergestellt. Verzweigte und vernetzte Polyester werden bei der Polykondensation von drei- oder mehrwertigen Alkoholen mit polyfunktionellen Carbonsäuren erhalten. Zu den Polyestern werden im Allgemeinen auch die Polycarbonate (Polyester der Kohlensäure) gerechnet.

Polyester des Typs I sind unter anderem Polyglykolsäuren, Polymilchsäuren, Polyhydroxybuttersäure, Poly(ε-caprolactone) und Polyhydroxybenzoesäuren.

Rein aliphatische Polyester des Typs II sind Polykondensate aus aliphatischen Diolen und Dicarbonsäuren, die unter anderem als Produkte mit endständigen Hydroxy-Gruppen als Polydiole für die Herstellung von Polyesterurethanen eingesetzt werden. Die mengenmäßig größte Bedeutung haben Polyester des Typs II aus aliphatischen Diolen und aromatischen Dicarbonsäuren insbesondere die Polyalkylenterephthalate mit Polyethylenterephthalat (PET), Polybutylenterephthalat (PBT), Poly(1,4-cyclohexandimethylenterephthalat)als wichtigste Vertreter.

Rein aromatische Polyester sind die Polyarylate, zu denen unter anderem Poly(4-hydroxybenzoesäure), Polykondensate aus Bisphenol A und Phosgen gehören.

Zusätzlich zu den bisher genannten Polyestern sind auch ungesättigte Polyester aus ungesättigten Dicarbonsäuren herstellbar, die als Polyesterharze, insbesondere als ungesättigte Polyesterharze (UP-Harze) technische Bedeutung erlangt haben. Als Polyester ist gemäß einer Ausführungsform der Erfindung das Polyethylenterephthalat (PET) bevorzugt.

Die Wandstärke der Verpackung aus Kunststoff wählt der Fachmann in Abhängigkeit des zu verpackenden Gegenstandes aus. Gemäß einer Ausführungsform der Erfindung weist die Verpackung eine Wandstärke von 1 bis 500 µm, bevorzugt von 20 bis 80 µm, besonders bevorzugt von 30 µm auf. Durch eine Wandstärke im vorstehend angegebenen Bereich wird sichergestellt, dass die Verpackung während der Lagerung oder dem Transport keinen Schaden nimmt und nicht durchlässig für Unsterilitäten, Bakterien und Schmutz wird.

Die Wand der erfindungsgemäßen Verpackung aus Kunststoff ist vorzugsweise einlagig aufgebaut.

Gemäß einer weiteren Ausführungsform der Erfindung besteht die Wand der Verpackung aus mehreren Lagen. Bevorzugt ist die Verpackung in diesem Falle dadurch gekennzeichnet, dass die innerste Lage im Wesentlichen Polyester umfasst, bzw. aus Polyester besteht. Der Vorteil bei der Verwendung von Polyester als Wandmaterial der erfindungsgemäßen Verpackung liegt darin, dass dieses Polymermaterial auch bei Temperaturen von etwa 60°C lediglich einen geringen Anteil an leichtflüchtigen Bestandteilen wie beispielsweise Weichmachern freisetzt. Hierdurch wird die Funktionalität des in der Verpackung befindlichen Instrumentes lang anhaltend gewährleistet, da sich nur verhältnismäßig geringe Mengen der freigesetzten Bestandteile auf der hydrophoben Beschichtung des Instrumentes absetzen können.
Eine besonders bevorzugte Ausführungsform sieht vor, dass die Verpackung ferner eine Testeinheit zur Beaufschlagung mit Körperflüssigkeit umschließt. Gemäß einer weiteren Ausführungsform der Erfindung sind das medizinische Instrument, vorzugsweise die Stechhilfe und Testeinheit in gesonderten Aufnahmezellen voneinander getrennt angeordnet. Die Zellen lassen sich beispielsweise einfach durch den Zwischenbereich zwischen den nicht weiter ausgeformten Folien realisieren. Durch die Trennung werden auch Vorteile dahingehend erreicht, dass die Stechhilfe ohne Schädigung der Testchemie unabhängig vorbehandelt, insbesondere sterilisiert und hydrophylisiert werden kann, und dass beim Stechvorgang keine Gefahr besteht, dass Testchemikalien in den Körper gelangen.

Erfindungsgemäß werden die medizinischen Instrumente in den zugeordneten Aufnahmezellen, die von der Verpackung gebildet werden, durch Bestrahlung, vorzugsweise über eine Maske zur Abschirmung der Testeinheiten sterilisiert. Da hydrophile Beschichtungen äußerst empfindlich sind und ferner beispielsweise Glucose-Teststreifen zusammen mit dem medizinischen Instrument verpackt werden, kommen als Sterilisationsmethoden besonders bevorzugt die Strahlungssterilisationsmethoden in Frage. Die Verpackung, welche als Verpackung des medizinischen Instruments dient, kennzeichnet sich folglich dadurch, dass diese durch β- oder γ-Strahlen, besonders bevorzugt durch β-Strahlen, sterilisiert ist.

Gemäß der Erfindung können als geeignete Folien für die Verpackung von hydrophil beschichteten medizinischen Instrumenten bzw. von mit hydrophilen Schichten beschichteten Instrumenten bevorzugt 30 µm dicke Folien, welche nach der β-Strahlen-Sterilisation einen Anteil von flüchtigen Bestandteilen von ≤ 0,1 µg/cm², bevorzugt von ≤ 0,01 1 µg/cm² aufweisen, identifiziert werden. Insbesondere zeigten bei Vergleichsversuchen Polyesterfolien - unabhängig vom Hersteller - sehr gute Ergebnisse bei der gaschromatographischen Analyse der flüchtigen Bestandteile. Derartige Folien wurden bis dahin größtenteils für die Verpackung von Lebensmitteln eingesetzt.

Die folgenden Beispiele und Vergleichsbeispiele erläutern die Erfindung, sind aber nicht zu ihrer Definition oder Beschränkung in irgendeiner Weise gedacht.

Verzeichnis der Abbildungen:
- Figur 1:: Kapillare und zugehöriges Bewertungsschema zum Testen einer erfindungsgemäßen Verpackung
- Figur 2:: Resultate eines Lagerungstests von mit SiO₂ hydrophil beschichteten Testplättchen in zwei verschiedenen Verpackungen.

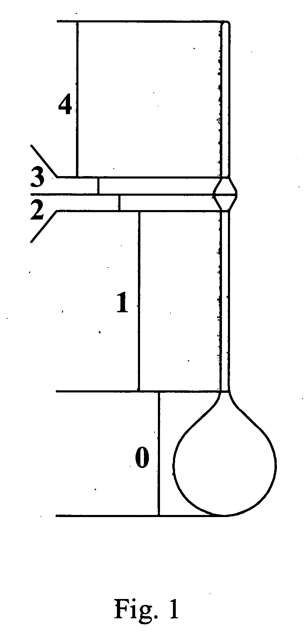

Figur 1 zeigt beispielhaft eine Kapillare. Die angegebene Skala von 0 bis 4 gibt das Füllverhalten der Kapillare wieder. Der Wert 1 auf dieser Skala steht für eine schlechte Befüllbarkeit der Kapillare, 4 für eine sehr gute Befüllbarkeit. Mit dem Wert 4 charakterisierte Kapillaren lassen eine beinahe vollständige oder vollständige Füllung der Kapillare mit Blut bzw. interstitieller Flüssigkeit zu.

Um herauszufinden, welche Folien beziehungsweise Materialien zur Lösung der erfindungsgemäßen Aufgabe geeignet sind, wurde ein Folienscreening durchgeführt. Dabei wurden die Anteile an flüchtigen Bestandteilen verschiedener Folien/Beutel mittels Headspace-Gaschromatographie ermittelt. Die entsprechenden Ergebnisse sind in der Tabelle 1 wiedergegeben und zeigen, dass Polyester-Folien mit wenig Additiven die geringsten Anteile an flüchtigen Bestandteilen nach der β-Sterilisation aufweisen.

**Tabelle 1: Anteil flüchtiger Bestandteile verschiedener Folien. Die mit "*" gekennzeichneten Werte geben die mit Hilfe eines Mikrometers bestimmte Dicke der jeweiligen Folie an.**

| Folie/Beutel | Hersteller | Dicke [µm] | Anteil an flüchtigen Bestandteilen [µg/cm²] | |
|---|---|---|---|---|
| | | | unbehandelt | nach β-Sterilisation |
| Melinex S (Polyester) | DuPont | 20* | < 0,01 | 0,01 |
| Hostaphan RN 23 MED (Polyester) | Mitsubishi | 23 | <0,01 | 0,01 |
| Hostaphan RN 19 (Polyester) | Mitsubishi | 19 | < 0,01 | 0,01 |
| Mylar 850 (Polyester) | DuPont | 20 | < 0,01 | 0,01 |
| Mylar 850 (Polyester) | DuPont | 30 | 0,01 | 0,01 |
| Mitsubishi RHS 12 (Polyester) | Mitsubishi | 12 | 0,01 | 0,01 |
| PA/PE | Medipack | 70* | 0,01 | 0,02 |
| Polialuvel Polyester/ALU/PE | Medipack | 90* | 0,01 | 0,05 |
| Teflon | DuPont | 50* | 0,01 | 0,06 |
| Hostaphan RHS 30 (Polyester) | Mitsubishi | 30 | 0,03 | 0,06 |
| Früh 24093 (PA/PE) | Früh | 90* | 0,03 | 0,06 |
| Alu/PE | Zewatener | 110* | 0,04 | 0,01 |
| Früh 24086 (PA/PE) | Früh | 90* | 0,16 | 0,16 |
| Topas 8007 F04 | Pütz Folien | 90 | 0,06 | 0,18 |
| Barex (Acrylnitril) | Früh | 50 | 0,11 | 0,31 |
| Topas 8007 PE Blend (COC/PE) | Pütz Folien | 70 | 0,28 | 0,41 |
| Siegelrandbeutel Polyester-O/ALU/PE, 12-9-75 | Sengewald Klinikprodukte | 96 | 0,4 | |

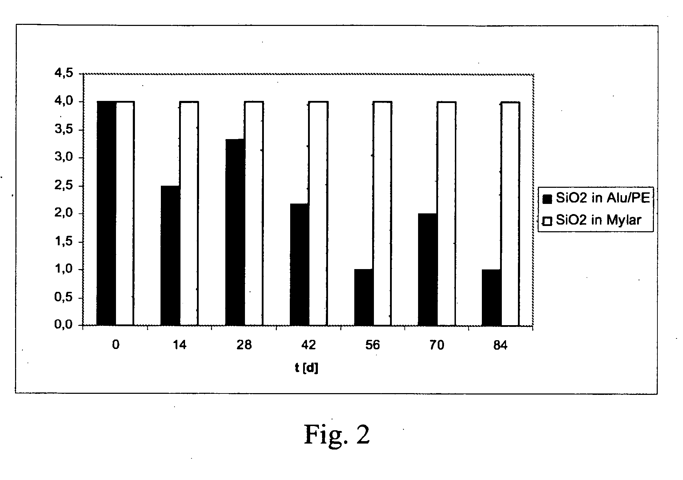

Anschließend wurde ein Experiment zur Ermittlung der Lagerstabilität einer hydrophilen Beschichtung in zwei verschiedenen Folien durchgeführt. 42 Testplättchen, welche Kapillaren, wie in Abb. 1 ersichtlich, aufwiesen, wurden mit einer hydrophilen Schicht aus SiO₂ beschichtet. Anschließend wurden 21 der Testplättchen in Alu/PE-Beuteln von Zewatener und 21 in Polyester-haltigen Mylar-Beuteln von DuPont verpackt. Im Anschluss daran wurden die verpackten Testplättchen β-sterilisiert (25 kgGy, 10 MeV) und über 12 Wochen bei 35°C gelagert. Im Abstand von zwei Wochen wurden die Plättchen getestet, indem jeweils 1,5 µl Blut ins Reservat der Kapillaren pipettiert und das Füllverhalten der jeweiligen Kapillare anhand des in Figur 1 gezeigten Beurteilungsschemas bewertet wurde. Die Resultate des Lagerungstests sind in Figur 2 dargestellt. In dieser Figur ist die Bewertung des Füllverhaltens der Kapillaren (Ranking von 1 bis 4) gegen die Lagerzeit nach der Sterilisation in Tagen aufgetragen. Wie aus Figur 2 hervorgeht, zeigen die Kapillaren der in Mylar-Beuteln (Anteil an flüchtigen Bestandteilen: 0,01 µg/cm²) verpackten Testplättchen über 84 Tage hinweg ein optimales Füllverhalten, während die in Alu/PE-Beuteln (Anteil an flüchtigen Bestandteilen: 0,06 µg/cm²) verpackten Testplättchen mit zunehmender Lagerungszeit sich immer schlechter füllten. Somit konnten die durch die gaschromatographische Untersuchung gefundenen Ergebnisse experimentell bestätigt werden.

Die gaschromatographische Prüfung auf die flüchtigen Bestandteile wurde wie folgt vorgenommen:

### Gerätepark, Zubehör und Chemikalien:

| | |
|---|---|
| Gaschromatograph: | Carlo Erba HRGC 5300 mit Head-Space, Autosampler im split-mode oder vergleichbares Gerät |
| | |
| Kapillare: | Firma Chrompack Best.N. 7761 oder vergleichbare Kapillare |
| | |
| Typ: | Wcot fused silica |
| Stationäre Phase: | CPSIL 8 CB |
| Filmdicke: | 1,12 µm |
| i.d.: | 0,32 mm |
| Länge: | 25 m |

### Glasgefäße und Arbeitsgeräte:

| | |
|---|---|
| Probengefäße: | 10 ml HS-Glas |

### Chemikalien:

| | |
|---|---|
| Toluol: Merck | Nr.1.09768.0005 |
| Schwefelkohlenstoff: Merck p.a. oder Chemikalien vergleichbarer Qualität | Nr. 1.02214.1000 |

### Teilprüfung auf flüchtige Bestandteile (GC)

### Standard:

20-25 mg Toluol werden in einen 50 ml-Kolben auf 0,1 mg genau eingewogen. In diesen wird 50 ml Schwefelkohlenstoff zupipettiert und ausgewogen. Ca. 10 mg dieser Lösung werden auf 0,1 mg genau in ein HS-Glas eingewogen und dieses sofort verschlossen.

Es werden zwei Standardlösungen hergestellt und von jeder Standardlösung 3 HS-Gläser eingewogen. Die Berechnung der flüchtigen Bestandteile erfolgt mit dem Mittelwert aller Standards.

Die Temperierung des Standards beträgt mindestens eine Stunde.

### Probenvorbereitung/Mustermenge:

Ein 50 cm² großes Folienstück der zu untersuchenden Probe wird klein geschnitten und in ein HS-Glas gegeben. Pro Muster wird mindestens eine Probe vorbereitet.

### Standzeit der Probe: 18 Stunden im HS-Bad vor der Analyse

### Chromatographische Bedingungen:

| Temperaturprogramm: | |
|---|---|
| T_{Anfang}: | 40°C, 5 min |
| Aufheizrate: | 10°C/min |
| T_{Ende}: | 280°C, 5 min |

### Teilprüfplan auf flüchtige Bestandteile (GC)

### Temperatur der Heizzonen:

### Mega 5300

| | |
|---|---|
| Injektor: | 250°C |
| Detektor: | 280°C |
| HS-Bad: | 100°C |
| HS-Spritze: | 100°C |

### Gasversorgung:

| | |
|---|---|
| Trägergas: | Helium |
| Vordruck: | 0,5 bar |
| Split: | 1:40 |
| Detektor: | gerätespezifische Einstellungen |

| FID | |
|---|---|
| Att.: | 7 |
| Mult.faktor: | 1 |

### Computersystem: Chrom Card oder vergleichbare Auswertemöglichkeit

### Injektionsvolumen: 1,25 ml

### Auswertung:

Die Auswertung erfolgt mit der externen Standardmethode. Dabei wir die Gesamtfläche aller flüchtigen Bestandteile zur Berechnung herangezogen und die Menge mit Hilfe der Toluolkonzentration und der Toluolpeakfläche berechnet. Aus allen Ergebnissen wird der Mittelwert gebildet.

## Patentansprüche

1. Verpackung aus Kunststoff umfassend zumindest eine Aufnahmezelle mit zumindest einem teilweise hydrophilen medizinischen Instrument zum Einstechen in Körpergewebe, das ausgewählt ist aus der Gruppe der Stechhilfen, Lanzetten, medizinischen Hilfsmittel und Mikronadeln, **dadurch gekennzeichnet, dass** das medizinische Instrument zumindest abschnittsweise eine hydrophile Beschichtung umfasst, wobei die Verpackung nach der Strahlungssterilisation einen Anteil an flüchtigen Bestandteilen von ≤0,1 µg/cm² aufweist, wobei die flüchtigen Bestandteile Bestandteile der Verpackung sind, welche sukzessive von dieser freigesetzt werden und wobei die flüchtigen Bestandteile mittels Headspace-Chromatographie charakterisiert und quantifiziert werden.

2. Verpackung aus Kunststoff nach dem vorstehenden Anspruch, umfassend Polyester.

3. Verpackung aus Kunststoff nach einem der vorstehenden Ansprüche, **dadurch gekennzeichnet, dass** die Verpackung eine Wandstärke im Bereich von 1 bis 500 µm aufweist.

4. Verpackung aus Kunststoff nach einem der vorstehenden Ansprüche, **dadurch gekennzeichnet, dass** diese mehrlagig aufgebaut ist.

5. Verpackung aus Kunststoff nach Anspruch 4, **dadurch gekennzeichnet, dass** die innerste Lage eine Lage aus Polyester ist.

6. Verpackung aus Kunststoff nach einem der vorstehenden Ansprüche, **dadurch gekennzeichnet, dass** die Verpackung eine Testeinheit zur Beaufschlagung mit Körperflüssigkeit umhüllt.

7. Verpackung aus Kunststoff nach einem der vorstehenden Ansprüche, **dadurch gekennzeichnet, dass** die Verpackung mittels β-Strahlung sterilisiert ist.

8. Verpackung aus Kunststoff nach einem der vorhergehenden Ansprüche, wobei die flüchtigen Bestandteile mittels des in den Beispielen und Vergleichsbeispielen beschriebenen Verfahrens charakterisiert und quantifiziert werden.

## Claims

1. Plastic package comprising at least one receiving compartment with at least one partially hydrophilic medical instrument for insertion into body tissue, which medical instrument is chosen from the group comprising puncturing aids, lancets, medical aids and microneedles, **characterized in that** the medical instrument comprises a hydrophilic coating at least in some sections, the package, after sterilization by radiation, having a proportion of volatile constituents of ≤ 0.1 µg/cm², the volatile constituents being constituents of the package that are successively released by the latter and the volatile constituents being **characterized** and quantified by means of headspace chromatography.

2. Plastic package according to the preceding claim, comprising polyester.

3. Plastic package according to one of the preceding claims, **characterized in that** the package has a wall thickness in the range of 1 to 500 µm.

4. Plastic package according to one of the preceding claims, **characterized in that** it has a multi-layer structure.

5. Plastic package according to Claim 4, **characterized in that** the innermost layer is a layer of polyester.

6. Plastic package according to one of the preceding claims, **characterized in that** the package encloses a test unit to which body fluid is to be applied.

7. Plastic package according to one of the preceding claims, **characterized in that** the package is sterilized by β radiation.

8. Plastic package according to one of the preceding claims, the volatile constituents being **characterized** and quantified by means of the method described in the examples and comparative examples.

## Revendications

1. Emballage en matériau synthétique comprenant au moins une cellule de réception comprenant au moins un instrument médical partiellement hydrophile destiné à être inséré dans le tissu corporel qui est choisi dans le groupe des aides au piquage, des lancettes, des matériels médicaux et des microaiguilles, **caractérisé en ce que** l'instrument médical comprend, au moins par sections, un revêtement hydrophile, l'emballage présentant après la stérilisation par un rayonnement une proportion de constituants volatils ≤ 0,1 µg/cm², les constituants volatils étant des constituants de l'emballage qui sont libérés successivement par ceux-ci et les constituants volatils étant **caractérisés** et quantifiés par chromatographie "Headspace".

2. Emballage en matériau synthétique selon la revendication précédente, comprenant du polyester.

3. Emballage en matériau synthétique selon l'une quelconque des revendications précédentes, **caractérisé en ce que** l'emballage présente une épaisseur de paroi dans la plage de 1 à 500 µm.

4. Emballage en matériau synthétique selon l'une quelconque des revendications précédentes, **caractérisé en ce qu'**il est formé de plusieurs couches.

5. Emballage en matériau synthétique selon la revendication 4, **caractérisé en ce que** la couche la plus interne est une couche en polyester.

6. Emballage en matériau synthétique selon l'une quelconque des revendications précédentes, **caractérisé en ce que** l'emballage entoure une unité de test destinée à recevoir du liquide corporel.

7. Emballage en matériau synthétique selon l'une quelconque des revendications précédentes, **caractérisé en ce que** l'emballage est stérilisé au moyen d'un rayonnement β.

8. Emballage en matériau synthétique selon l'une quelconque des revendications précédentes, les constituants volatils étant **caractérisés** et quantifiés au moyen du procédé décrit dans les exemples et les exemples comparatifs.
